(19) Europäisches Patentamt
European Patent Office
Office européen des brevets

(11) Publication number: **0 160 578 B1**

# (12) EUROPEAN PATENT SPECIFICATION

(45) Date of publication of patent specification: **23.11.89**

(51) Int. Cl.⁴: **C 07 D 471/04** // A61K31/435, C07D213/61, C07D213/80 ,(C07D471/04, 221:00, 221:00)

(21) Application number: **85400270.6**

(22) Date of filing: **15.02.85**

## (54) 1,8-Naphthyridine derivatives.

(30) Priority: **17.02.84 JP 28278/84**
**19.03.84 JP 53159/84**

(43) Date of publication of application:
**06.11.85 Bulletin 85/45**

(45) Publication of the grant of the patent:
**23.11.89 Bulletin 89/47**

(84) Designated Contracting States:
**BE CH DE FR GB IT LI NL SE**

(56) References cited:
**EP-A-0 049 355**
**EP-A-0 078 362**
**EP-A-0 106 489**
**EP-A-0 132 845**
**EP-A-0 153 163**
**EP-A-0 153 828**
**FR-A-2 500 833**

(73) Proprietor: **DAIICHI SEIYAKU CO. LTD.**
**14-10, Nihonbashi 3-chome**
**Chuo-ku Tokyo 103 (JP)**

(72) Inventor: **Hayakawa, Isao c/o Daiichi Seiyaku Research Institute 16-13 Kitakasai 1-chome Edogawa-ku Tokyo 103 (JP)**
Inventor: **Imamura, Masazumi c/o Daiichi Seiyaku Research Institute 16-13 Kitakasai 1-chome Edogawa-ku Tokyo 103 (JP)**
Inventor: **Kanaya, Naoaki c/o Daiichi Seiyaku Research Institute 16-13 Kitakasai 1-chome Edogawa-ku Tokyo 103 (JP)**

(74) Representative: **Hirsch, Marc-Roger**
**Cabinet Hirsch 34 rue de Bassano**
**F-75008 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 160 578 B1

**Description**

Field of the Invention

The present invention relates to a novel 1,8-naphthyridine derivatives presenting outstanding antibacterial activity as well as to its pharmacologically acceptable salts; it also relates to a process for their preparation.

Description of the Prior Art

Japanese patent application n° 131346/82 filed on July 29, 1982 and laid open to public inspection on May 6, 1983 under Disclosure No. 74667/83 discloses bacteriocidal 1-cyclopropyl-6-fluoro-1,4-dihydro-4-oxo-7-piperazinoquinoline-3-carboxylic acids.

Furthermore, Japanese patent application No. 136 224/81 filed on September 1, 1981 and laid open to public inspection on May 15, 1982 under Disclosure No. 77683/82 discloses antibacterial 7-piperazino-1-cyclopropyl-4-oxo-1,4-dihydro-1,8-naphthyridine-3-carboxylic acid compounds.

EP—A—153828 as well as EP—A—0153163 concern 1-cyclopropyl-6,8-difluoro-1,4-dihydro-4-oxo-3-quinoline carboxylic acids derivatives as well as 1-cyclopropyl-1,4-dihydro-6-fluoro-4-oxo-1,8 naphthyridine. These derivatives differ from the compounds according to the present invention.

Brief summary of the invention

The present invention concerns novel 1,8-naphthyridine derivatives represented by the general formula (I):

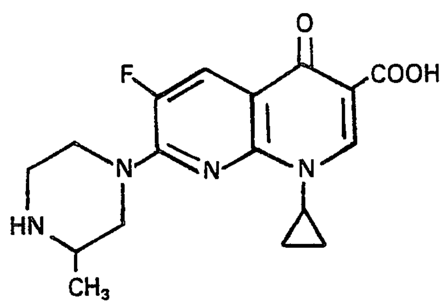

and its pharmacologically acceptable salts, and a process for their preparation. The said 1,8-naphthyridine derivatives (I) and its pharmacologically acceptable salts are useful as antibacterial agents. The process comprises reacting 7-chloro-1-cyclopropyl-6-fluoro-1,4-dihydro-4-oxo-1,8-naphthyridine-3-carboxylic acid with the cyclic amine is 2-methyl-1-piperazinyl.

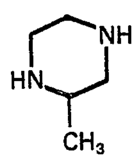

The 3-carboxylic acid is prepared from the known 2,6-dihydroxy-3-fluoropyridine-5-carboxamide or 2,6-dichloro-3-fluoropyridine-5-carbonitrile.

Thus, it is an object of the present invention to provide novel compound and its salts presenting outstanding antibacterial activity.

Another object of the present invention is to provide a process for the preparation of these compounds.

These and other objects and advantages of the present invention will become apparent from reading through the following description.

Detailed description of the preferred embodiments

The term "pharmacologically acceptable salts" include salts with inorganic acids such as hydrochloric acid and sulfuric acid, with organic acids such as methanesulfonic acid, acetic acid and gluconic acid and with alkali or alkaline earth metals including sodium, potassium and calcium.

Compound (I) may be in the form of hydrates.

The synthesis of the compound of the present invention may be exemplified by the following reaction scheme:

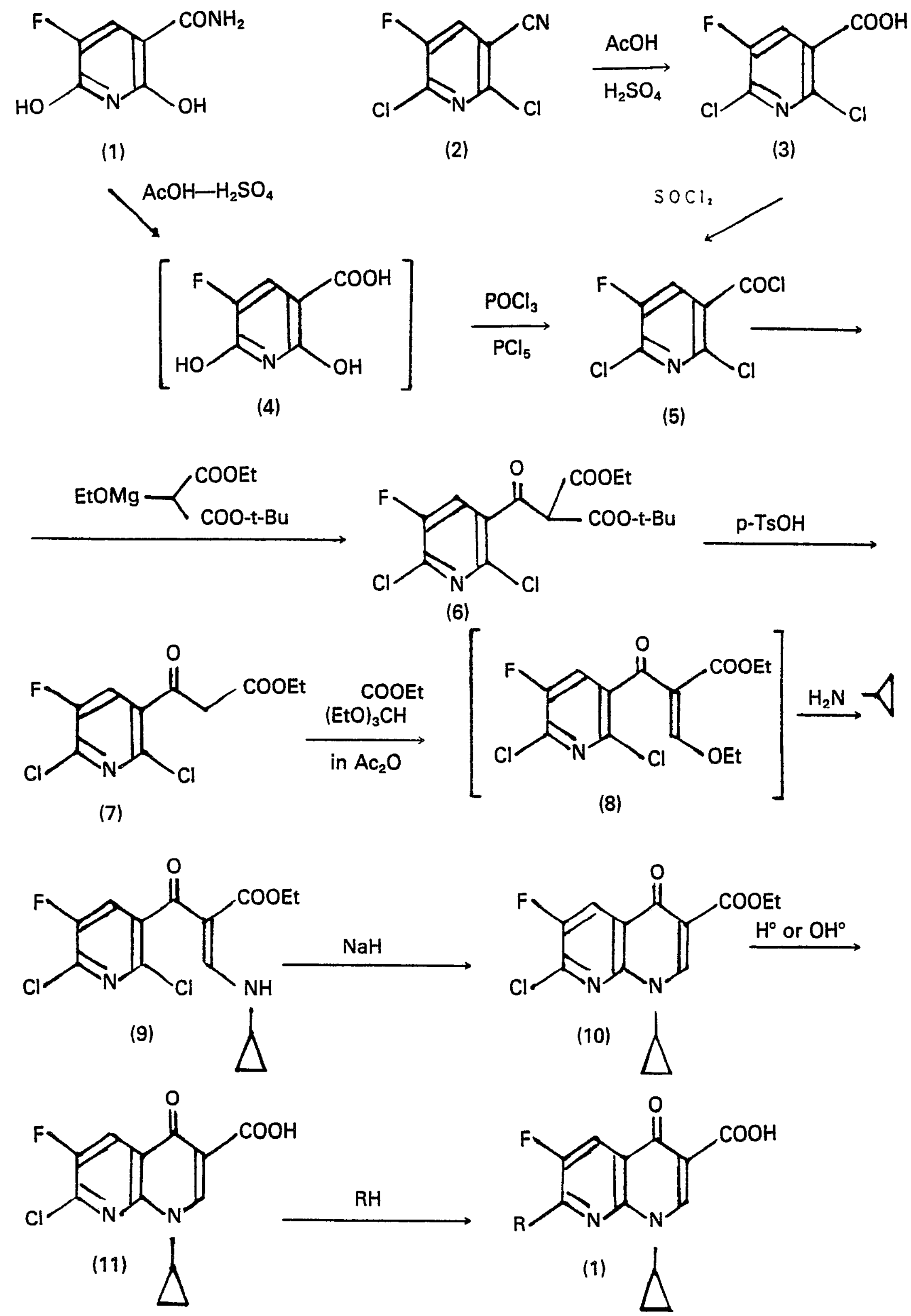


In the above reaction scheme, the known 2,6-dihydroxy-3-fluoropyridine-5-carboxamide (1) is hydrolyzed with sulfuric acid-acetic acid to yield a carboxylic acid (4) which is, without further purification, treated with phosphorus oxychloride-phosphorus pentachloride to produce 2,6-dichloro-3-fluoropyridine-5-carbonyl chloride (5). Alternatively, this acid chloride (5) can be obtained by hydrolyzing the known 2,6-dichloro-3-fluoropyridine-5-carbonitrile (2) to the corresponding carboxylic acid (3) followed by reflux of the latter in thionyl chloride and benzene. The acid chloride (5) is added to ethyl t-butyl ethoxy-magnesium-malonate, which is synthesized from ethoxy-magnesium ethoxide and ethyl t-butyl malonate, to yield ethyl t-butyl

2,6-dichloro-5-fluoronicotinoylmalonate (6) which is then refluxed with a catalytic amount of p-toluenesulfonic acid in benzene over a period of 3-5 hours to yield ethyl 2,6-dichloro-5-fluoronicotinoylacetate (7) (a mixture of keto and enol forms). This ester is reacted with ethyl orthoformate in acetic anhydride to produce ethyl 3-ethoxy-2-(2,6-dichloro-5-fluoronicotinoyl)-acrylate (8). This ester is then dissolved in dichloromethane without further purification. Cyclopropylamine at room temperature is added to the resulting solution to produce the corresponding 3-cyclopropylamino compound (9). This product is dissolved in anhydrous dioxane followed by addition of sodium hydride. The resulting mixture is then refluxed for 5—30 minutes and thereafter the product is purified by chromatography on silica gel. Thus, ethyl 7-chloro-1-cyclopropyl-6-fluoro-1,4-dihydro-4-oxo-1,8-naphthyridine-3-carboxylate (10) is obtained. This compound (10) is converted to the corresponding carboxylic acid (11) by acid or alkaline hydrolysis and then reacted with the above cyclic amine consisting in 2-methyl-1-piperazinyl to produce the object compound (I).

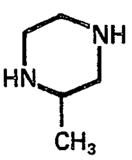

This reaction can be carried out in a solvent such as dimethylsulfoxide, dimethyl-formamide, pyridine or 3-methoxybutanol. Compound (I) may be produced over 30 minutes to five hours at a temperature comprised between room temperature and 120°C, preferably at a temperature within the range of 40—100°C, and usually they may be produced from between 30 minutes and 2 hours.

The antibacterial activity of the compounds of the present invention was determined by the standard method of the Japan Society of Chemotherapy: according to the dilution method in Muller-Hinton Bouillon, $10^6$/ml of bacteria were seeded and incubated at 37°C for 18 hours. The results obtained by the method mentioned herein-above are summarized in the following table:

TABLE

Minimal Inhibitory Concentration (MIC) (μg/ml)

| | No. 1 | Control Compound |
|---|---|---|
| E. coli, N I H J | ≦0.05 | 0.20 |
| Sh. flexneri, 2a5503 | ≦0.05 | 0.20 |
| Pr. vulgaris, 3167 | ≦0.05 | 0.20 |
| Pr. mirabilis, 1287 | 0.10 | 0.20 |
| Ps. marcescens, 13001 | ≦0.05 | 0.20 |
| Ser. aeruginosa, 2063 | 0.20 | 0.78 |
| Ps. aeruginosa, 2128 | 0.20 | 0.78 |
| Ps. aeruginosa, 2131 | 0.10 | 0.20 |
| Ps. cepacia, II D1340 | 0.39 | 50.0 |
| Ps. maltophilia, II D1275 | 0.39 | 0.78 |
| S. aureus, Smith | 0.39 | 0.78 |
| S. epidermis, 56556 | 0.78 | 0.78 |
| Str. pyrogenes, G—36 | 1.56 | 25.0 |
| Str. faecalis, ATCC19433 | 3.13 | 6.25 |

Control compound:

1-ethyl-6-fluoro-7-(1-piperazinyl)-1,4-dihydro-4-oxo-1,8-naphthyridine-3-carboxylic acid (Example 2 of Japanese Patent Application Publication No. 10109/1982).

As shown in the above table, the antibacterial activity of the compound of the present invention is superior to that of the control compound. Furthermore, it shows significantly high oil solubility as compared with the corresponding quinoline derivatives, and, moreover, is expected to be satisfactorily absorbed through the intestinal tract and to show a high concentration in blood.

The compound of the present invention is useful as antibacterial agents for the treatment of various infectious diseases such as urinary tract infections or infections in respiratory organs of mammals including humans. Normally this compound is administered orally, but it can also be administered by injection or can be used by external application depending upon the type of disease to be treated.

Orally the compound of the present invention can be administered at a dosage between 100 mg to 1000 mg, normally between 100 mg to 600 mg, per day for adults, in the form of various pharmaceutical preparations such as tablets, capsules, powders, granules, syrup and the like which are well known in the prior art. Preparations of the compound of the present invention suitable for injection or external application can also be prepared by techniques known in the art. For example, they can be prepared by methods known per se using suitable diluents, binders, excipients, disintegrators, coating agents and the like.

The acute toxicity of the compound of the present invention (I) was 200 or more mg/kg in mice (i.v.).

The present invention will be explained herein-below in more detail with reference to the Reference Example and Examples given below. Unless otherwise indicated, all parts, percents, ratios and the like are by weight.

Reference Example

(a) 12 g of 2,6-dichloro-3-fluoropyridine-5-carbonitrile (2) was added to a mixture of 60 ml of acetic acid, 5.8 ml of water and 5.8 ml of conc. sulfur acid followed by reflux for 16 hours. After the reaction, 200 ml of water was added to the reaction mixture which was then submitted to 3 successive extractions with 150 ml portions of ethyl acetate. The extracts were washed with 100 ml of a saturated aqueous saline solution, dried over sodium sulfate, and the solvent was distilled off to yield 6.8 g of 2,6-dichloro-3-fluoro-pyridine-5-carboxylic acid (3) as crystalline powders:

NMR: $CDCl_3$ δ ppm

8.10 (1H, d, J=8.0Hz, aromatic *H*)

9.92 (1H, broad S, —COO*H*)

(b) 5 ml of thionyl chloride and 60 ml of benzene were added to 3.9 g of the carboxylic acid (3) from step (a), and the resulting mixture was refluxed for 1 hour. Thereafter, the mixture was cooled, the solvent was distilled off, and then benzene was added to the resulting residue followed by stirring. The mixture was subjected to two successive treatments for removing the supernatant benzene to yield an acid chloride (5) as an oily material. This material was dissolved in 20 ml of ether and then the resulting solution was gradually added dropwise to another solution which was separately prepared by refluxing 3.67 g of ethyl t-butyl malonate and 2.2 g of magnesium ethoxide in 40 ml of ether for 1 hour and then cooling the mixture to room temperature. After the addition, the resulting mixture was refluxed for 15 minutes, and then the solution was allowed to cool to room temperature followed by addition of water thereto. The pH of the solution was adjusted to lower than 4 with sulfuric acid, and it was partitioned with ether. The aqueous layer was then submitted to three successive extractions with 100 ml portions of ether. The combined ether extracts were washed with a saturated aqueous saline solution and dried over sodium sulfate. The removal of the ether gave 3.4 g of ethyl t-butyl 2,6-dichloro-5-fluoronicotinoylmalonate (6) as an oily material.

NMR: $CDCl_3$ δ ppm

1.48, 1.58 (two t-butyls)

7.49 (1H, d, pyridine *H*)

(c) 3.1 g of 2,6-dihydroxy-3-fluoropyridine-5-carboxamide (1) (or 2,6-dihydroxy-5-fluoronicotinamide) was added to a mixture of 10 ml of acetic acid, 1 ml of water and 4 ml of sulfuric acid, and the resulting mixture was refluxed for 24 hours. Thereafter the mixture was cooled and the solvent was distilled off under reduced pressure, 10 ml of ice water was added to the residue, and the mixture was neutralized by addition of sodium bicarbonate thereto and then extracted with excess chloroform. After drying over sodium sulfate the solvent was distilled off, and the residue was, without further purification, added to a mixture of 5 ml of phosphorus oxychloride and 1 g of phosphorus pentachloride. The resulting mixture was refluxed for 3 hours and then cooled, and thereafter the solvent was distilled off under reduced pressure. The resulting residue was stirred after addition of 20 ml of benzene thereto and then subjected to three successive treatments for removing the supernatant benzene to yield 2.2 g of 2,6-dichloro-3-fluoropyridine-5-carbonyl chloride (5).

(d) 150 mg of p-toluenesulfonic acid and 100 ml of dry benzene were added to 3.4 g of ethyl t-butyl 2,6-dichloro-5-fluoronicotinoylmalonate (6). The resulting mixture was refluxed for 3 hours, and then the solvent was distilled off under reduced pressure. The residue was purified by chromatography on silica gel (50 g). Thus, 2.3 g of ethyl 2,6-dichloro-5-fluoronicotinoylacetate (7) was obtained from the benzene extracts.

NMR: $CDCl_3$ δ ppm

1.35 (3H, t, J=7Hz, $—CH_2CH_3$)

4.28 (2H, q, J=7Hz, $—CH_2CH_3$)

4.08 and 5.80 (enol)

2H ( 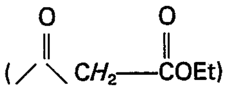 )

7.78 (1H, d, J=8Hz, pyridine *H*)

(e) 1.3 g of ethyl orthoformate and 40 ml of acetic anhydride were added to 2.2 g of the β-keto ester (7) from the step (d), and the mixture was heated to reflux for 15 minutes. Then the solvent was distilled off and 30 ml of dichloromethane was added to the crude ethoxymethylene compound (8) thus obtained. To the resulting mixture under cooling with ice and stirring was added dropwise 5 ml of a solution of 500 mg of cyclopropylamine in dichloromethane. The mixture was stirred for 20 minutes at room temperature, and the solvent was distilled off. The resulting residue was purified by chromatography on silica gel (20 g) (eluant 3%-ethyl acetate/benzene 20 g) to give 1.3 g of ethyl 3-cyclopropylamino-2-(2,6-dichloro-5-fluoro-nicotinoyl)-acrylate (9).

NMR: $CDCl_3$ δ ppm

0.8—1.0 (4H, m, > N 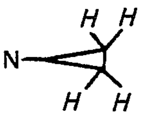 )

1.06 (3H, t, J=7Hz, $—CH_2CH_3$)

3.00 (1H, m, > N 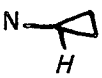 )

4.02 (2H, q, J=7Hz, $—CH_2CH_3$)

8.21 (1H, d, J=13Hz, pyridine *H*)

(f) 690 mg of the compound (9) from the step (e) was added to 20 ml of a suspension of 105 mg of sodium hydride (50% dispersion in oil) in dioxane, and the mixture was refluxed for 10 minutes. The reaction solution was red colored. The solution was allowed to cool to room temperature, poured into 50 ml of ice water and then made acidic (pH < 3) with dilute hydrochloric acid. The mixture was extracted with three portions each of 50 ml of chloroform and dried over sodium sulfate, and then the residue was purified through a column of silica gel (10 g). Thus, ethyl 7-chloro-1-cyclopropyl-6-fluoro-1,4-dihydro-4-oxo-1,8-naphthyridine-3-carboxylate (10) was obtained as chloroform extracts. Recrystallization from ethanol gave 270 mg of colorless needles of the compound (10) (mp.: 175—176.5°C).

NMR: $CDCl_3$ δ ppm

0.9—1.4 (4H, m, > N 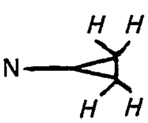 )

1.40 (3H, t, J=7Hz, $—CH_2CH_3$)

3.64 (1H, m, – N 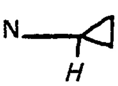 )

4.40 (2H, q, J=7Hz, $—CH_2CH_3$)

8.82 (1H, d, J=8Hz, aromatic $C_5—H$)

8.64 (1H, s, $C_2—H$)

Analysis ($C_{14}H_{12}ClFN_2O_3$):

| | C | H | N |
|---|---|---|---|
| calculated | 54.12 | 3.89 | 9.01 |
| found | 54.31 | 3.87 | 9.00 |

(g) 350 mg of the ester (8) from the step (f) was added to 20 ml of a mixture of acetic acid and hydrochloric acid (1:1), and the resulting mixture was refluxed for 1.5 hours. The reaction mixture was concentrated under reduced pressure to between 5 and 10 ml and 20 ml of water was added to the concentrate. The crystals were collected by filtration and washed successively with water, ethanol and ether. Recrystallization from ethanol gave 240 mg of 7-chloro-1-cyclopropyl-6-fluoro-1,4-dihydro-4-oxo-1,8-naphthyridine-3-carboxylic acid (11) melting at 223—224°C.

NMR: $CDCl_3$ δ ppm

1.0—1.4 (4H, m, > N 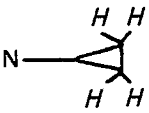 )

3.8—3.95 (1H, m, > N 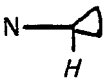 )

8.48 (1H, d, J=8Hz, $C_5$—*H*

8.96 (1H, s, $C_2$—*H*)

Analysis ($C_{12}H_8FClN_2O_3 . 1/4\ H_2O$)

| | C | H | N |
|---|---|---|---|
| calculated | 50.19 | 2.98 | 9.75 |
| found | 50.13 | 3.18 | 9.50 |

Example 1

130 mg of 2-methylpiperazine and 5 ml of pyridine were added to 100 mg of 7-chloro-1-cyclopropyl-6-fluoro-1,4-dihydro-4-oxo-1,8-naphthyridine-3-carboxylic acid (11) and the mixture was stirred at an external temperature of 60°C for 30 minutes. Then the solvent was distilled off under reduced pressure, and 2 ml of ethanol was added to the residue to produce crystallization. The crystals were collected by filtration and washed successively with ethanol and ether. After being dried, the resulting crude crystals were suspended in 10 ml of ethanol and then dissolved therein by addition of excess conc. aqueous ammonia and then treated with activated carbon. The mixture was concentrated to remove the ammonia thereby giving as transparent crystals 40 mg of 1-cyclopropyl-6-fluoro-7-(3-methyl-1-piperazinyl)-1,4-dihydro-4-oxo-1,8-naphthyridine-3-carboxylic acid (mp.: 236—239°C).

NMR: DMSO-$d_6$ δ ppm

1.0—1.4 (7H, m, N 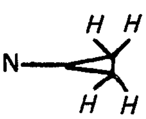 & 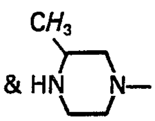 )

2.7—4.6 (7H, m, piperazine *H*)

8.06 (1H, d, J=14Hz, $C_5$—*H*)

8.62 (1H, s, $C_2$—*H*)

Analysis ($C_{17}H_{19}FN_4O_3$)

| | C | H | N |
|---|---|---|---|
| calculated | 58.95 | 5.52 | 16.18 |
| found | 58.66 | 5.78 | 15.91 |

The following is an example of preparation for oral administration containing the compound of the present invention (I).

Capsules

| | | |
|---|---|---|
| compound (I) | | 100.0 mg |
| corn starch | | 23.0 mg |
| CMC calcium | | 22.5 mg |
| hydroxypropylmethyl cellulose | | 3.0 mg |
| magnesium stearate | | 1.5 mg |
| | Total | 150.0 mg per capsule |

**Claims**

1. 1 - cyclopropyl - 6 - fluoro - 7 - (3 - methyl - 1 - piperazinyl) - 1,4 - dihydro - 4 - oxo - 1,8 - naphthyridine - 3 - carboxylic acid represented by the following formula:

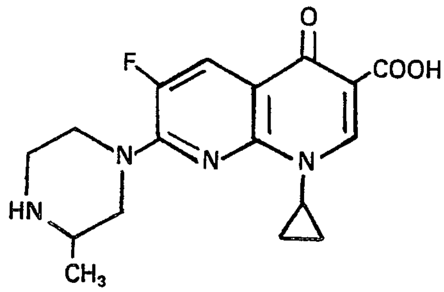

2. A process for the preparation of 1-cyclopropyl-6-fluoro-7-(3-methyl-1-piperazinyl)-1,4-dihydro-4-oxo-1,8-naphthyridine-3-carboxylic acid represented by the following formula:

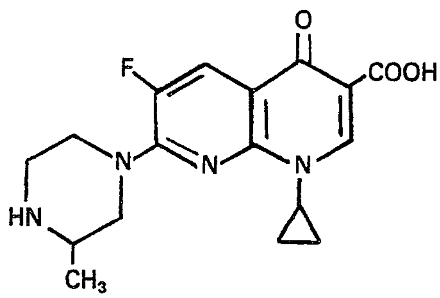

which consists reacting 7-chloro-1-cycloporopyl-6-fluoro-1,4-dihydro-4-oxo-1,8-naphthyridine-3-carboxylic acid with 2-methylpiperazine of the following formula

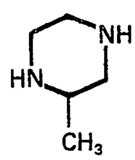

**Patentansprüche**

1. 1 - cyclopropyl - 6 - fluoro - 7 - (3 - méthyl - 1 - piperazinyl) - 1,4 - dihydro - 4 - oxo - 1,8 - naphtyridine - 3 - Kohlensäure mit dem Formel

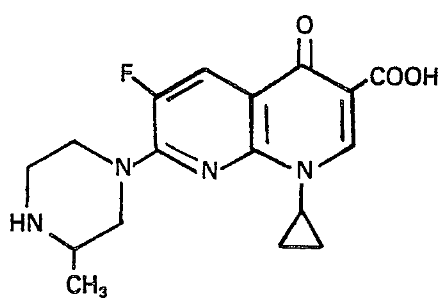

2. Verfahren Zur Vorbereitung von 1-cyclopropyl-6-fluoro-7-(3-méthyl-1-piperazinyl)-1,4-dihydro-4-oxo-1,8-naphtyridine-3-Kohlensäure mit dem Formel

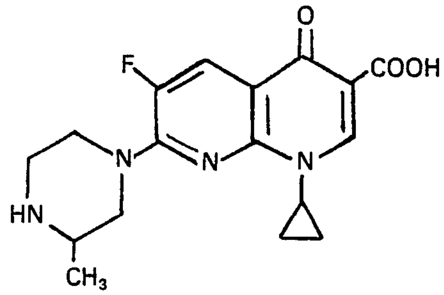

dadurchdgekumzeichnet dass man die 7-chloro-1-cyclopropyl-6-fluoro-1,4-dihydro-4-oxo-1,8-naphtyridine-3-Kohlensäure mit 2-methylpiperazin der folgenden formel:

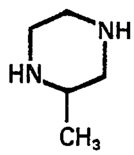

umsetzt.

**Revendications**

1. Un dérivé d'acide l-cyclopropyl-6-fluoro-7-(3-méthyl-1-pipérazinyl)-1,4-dihydro-4-oxo-1,8-naphtyridine-3-carboxylique représenté par la formule:

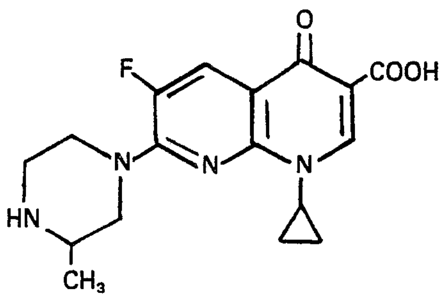

2. Un procédé de préparation du dérivé d'acide 1-cyclopropyl-6-fluoro-7-(3-méthyl-1-pipérazinyl)-1,4-dihydro-4-oxo-1,8-naphtyridine-3-carboxylique représenté par la formule:

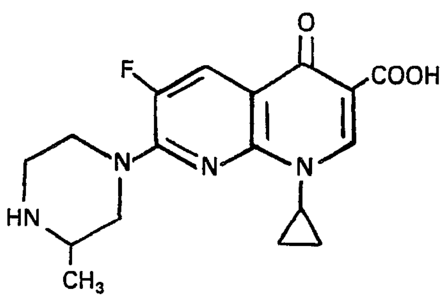

constant en la réaction de l'acide 7-chloro-1-cyclopropyl-6-fluoro-1,4-dihydro-4-oxo-1,8-naphtyridine-3-carboxylique avec la méthylpipérazine présentant la formule suivante: